# EUROPEAN PATENT APPLICATION

(11) **EP 4 821 896 A1**
(43) Date of publication of application: **14.10.2026**
(21) Application number: 24912033.8
(22) Date of filing: 06.11.2024
(51) Int. Cl.: C02F 3/12, C12N 1/20

(54) **ACTIVATED SLUDGE-BASED DECOMPOSITION TREATMENT METHOD, WASTEWATER TREATMENT DEVICE, AND ACTIVATED SLUDGE**

(30) Priority: 26.12.2023 JP 2023220108
(71) Applicant: Jeplan, Inc., Kawasaki-shi, Kanagawa 210-0867 (JP)
(72) Inventor: AKIHO, Satoru, Kawasaki-shi, Kanagawa 210-0867 (JP); KAWAI, Yuki, Kawasaki-shi, Kanagawa 210-0867 (JP)
(74) Representative: Hoffmann Eitle
(86) International application number: PCT/JP2024/039478
(87) International publication number: WO 2025/142140

(57) **Abstract**

Summary [Problem] To provide: an activated sludge-based decomposition treatment method for a substance to be treated that contains at least one from among phthalic acids and esters thereof and oligomers of the preceding; a wastewater treatment device; and an activated sludge.

[Solution] An activated sludge-based decomposition treatment method is provided according to one embodiment of the present invention. This decomposition treatment method comprises: a first step for preparing an activated sludge in a wastewater treatment device that treats wastewater generated in a process of lowering the molecular weight of an aromatic polyester; and a second step for decomposing the substance to be treated in a mixed liquid obtained by mixing the activated sludge and a liquid to be treated containing a substance to be treated that contains at least one from among phthalic acids and esters thereof and oligomers of the preceding. Potassium ion is added to the activated sludge in the second step.

## Description

### Technical Field

The present invention relates to an activated sludge-based decomposition treatment method, more particularly to an activated sludge-based decomposition treatment method for a substance to be treated including at least one selected from phthalic acid, phthalic acid esters, and oligomers thereof, a wastewater treatment device, and activated sludge.

### Background Art

With growing awareness of environmental issues, standards for factory wastewater are becoming stricter around the world. In the construction of new factories, it is required to provide evidence that wastewater treatment can satisfy regulatory standards. Thus, wastewater treatment is now positioned as one of the important technologies forming part of the manufacturing process.

Polyethylene terephthalate (hereinafter also referred to as "PET"), which is used in beverage bottles and polyester fibers, may generate wastewater during its manufacturing process and recycling process. Such wastewater may contain high concentrations of PET degradation products, which are difficult to sufficiently decompose using conventional activated sludge.

For example, wastewater discharged in a chemical recycling process of PET contains bis(2-hydroxyethyl) terephthalate (hereinafter also referred to as "BHET"), which is difficult to decompose using conventional activated sludge. In such a case, it is necessary to outsource wastewater treatment at additional expense, or to adjust plant operations, resulting in a significant impact on factory operating costs.

Although it has been reported that Enterobacter sp. has been isolated as a microorganism capable of decomposing BHET, only about 30% of 2000 mg/L (about 8 mM) of BHET can be decomposed even after 120 hours (see Non-Patent Document 1). Additionally, when such isolated microorganisms are used for wastewater treatment, there is a problem in that the activity of the isolated microorganisms cannot be maintained because they are preyed upon by activated sludge that is mixed therein. Prior Art Documents

### [Non-Patent Documents]

[Non-Patent Document 1] Lequan Qiu et al., J Basic Microbiol. 2020; 60:699-711

### Summary of Invention

### Problems to be Solved by Invention

In view of the above-described circumstances, the present invention provides an activated sludge-based decomposition treatment method for a substance to be treated including at least one selected from phthalic acids, esters thereof, and oligomers thereof, a wastewater treatment device, and activated sludge.

### Means for Solving Problems

According to one aspect of the present invention, an activated sludge-based decomposition treatment method is provided. The decomposition treatment method includes: a first step of preparing activated sludge in a wastewater treatment device configured to treat wastewater generated in a process of reducing the molecular weight of an aromatic polyester; and a second step of decomposing a substance to be treated in a mixed liquor obtained by mixing the activated sludge with a liquid to be treated containing the substance to be treated, the substance to be treated including at least one selected from phthalic acids, esters thereof, and oligomers thereof. In the second step, potassium ions are added to the activated sludge.

According to such an aspect, it is possible to rapidly treat a substance to be treated including at least one selected from phthalic acids, esters thereof, and oligomers thereof, which has conventionally been difficult to decompose. Detailed Description

Hereinafter, preferred embodiments of an activated sludge-based decomposition treatment method, a wastewater treatment device, and activated sludge of the present invention will be described in detail.

In the present specification, the term "polyester" refers to a polymer compound having an ester bond in a main chain. Additionally, in the present specification, the term "aromatic polyester" refers to a polyester containing an aromatic component as a repeating unit.

The content of such repeating units is about 50% by mass or more and about 100% by mass or less (preferably about 70% by mass or more and about 100% by mass or less, more preferably about 90% by mass or more and about 100% by mass or less, and still more preferably about 95% by mass or more and about 100% by mass or less), based on the total amount of the compound.

Examples of the aromatic polyester include PET, and PET containing 95% by mass or more of ethylene terephthalate repeating units is preferred.

Aromatic polyesters can be produced by polycondensation of a dicarboxylic acid component and a diol component.

For example, PET can be produced by using terephthalic acid as the dicarboxylic acid component and ethylene glycol as the diol component.

Examples of dicarboxylic acid components other than terephthalic acid include aromatic dicarboxylic acids such as phthalic acid, isophthalic acid, diphenyl dicarboxylic acid, diphenoxyethane dicarboxylic acid, and 2,5-naphthalene dicarboxylic acid, or derivatives thereof; and aliphatic dicarboxylic acids such as succinic acid, adipic acid, azelaic acid, sebacic acid, and decanedicarboxylic acid, or derivatives thereof.

Examples of diol components other than ethylene glycol include diethylene glycol, trimethylene glycol, tetramethylene glycol, propylene glycol, pentamethylene glycol, hexamethylene glycol, and decamethylene glycol.

The activated sludge-based decomposition treatment method of the present invention includes: a first step of preparing activated sludge in a wastewater treatment device configured to treat wastewater generated in a process of reducing the molecular weight of an aromatic polyester; and a second step of decomposing a substance to be treated in a mixed liquor obtained by mixing the activated sludge with a liquid to be treated containing the substance to be treated, the substance to be treated including at least one selected from phthalic acids, esters thereof, and oligomers thereof.

Hereinafter, each step will be described in sequence.

### First Process

[1] First, in the first step, activated sludge in a wastewater treatment device configured to treat wastewater generated in a process of reducing the molecular weight of an aromatic polyester is provided. The activated sludge may be used as it is in an activated sludge tank of the wastewater treatment device, or may be collected and stored in another storage tank (storage section) for use.

Here, the form of the aromatic polyester whose molecular weight is to be reduced is not particularly limited, and examples thereof include fibrous, particulate, flake, pellet, film, lump, and bottle forms, or a mixture thereof.

Examples of the process of reducing the molecular weight of the aromatic polyester include a chemical recycling process for chemically decomposing PET, such as methanolysis, glycolysis, and hydrolysis. In the chemical recycling process of PET, PET is depolymerized using ethylene glycol to produce an intermediate, i.e., BHET. Next, in order to increase purity, BHET is subjected to a purification step (distillation and recrystallization). Wastewater discharged during recrystallization contains high concentrations of related substances, such as BHET as a main component, mono(2-hydroxyethyl) terephthalate (hereinafter also referred to as "MHET"), and terephthalic acid (hereinafter also referred to as "TPA")
The wastewater treatment device treats wastewater generated in this chemical recycling process, which is namely wastewater mainly containing BHET.

In the present invention, activated sludge in such a wastewater treatment device configured to treat wastewater is used. Therefore, such activated sludge is capable of decomposing PET degradation products mainly containing BHET. That is, in the present invention, the liquid to be treated, which is subjected to decomposition treatment using activated sludge, mainly contains BHET.

Here, activated sludge is a general term for "living" suspended organic sludge containing an aerobic microbial consortium that has been artificially and industrially cultivated and grown. Activated sludge is widely used as a means of purifying wastewater or sewage in sewage treatment plants, human waste treatment plants, septic tanks, and the like.

However, even when such activated sludge is used, decomposition products of aromatic polyesters such as BHET cannot be sufficiently decomposed; therefore, in wastewater (waste) treated with activated sludge generated in a chemical recycling process of PET, decomposition products of aromatic polyesters such as BHET usually remain.

### [2] Second Process

Next, in the second step, a substance to be treated is decomposed in a mixed liquor obtained by mixing the activated sludge with a liquid to be treated containing the substance to be treated, the substance to be treated including at least one selected from phthalic acids, esters thereof, and oligomers thereof.

Examples of the substance to be treated include, in addition to BHET, TPA, MHET, dimethyl terephthalate (DMT), isophthalic acid (IPA), phthalic acid, and the like. Additionally, examples of the oligomers include polymers containing, as constituent units, phthalic acids or esters thereof in an amount of about 2 to about 10 units.

These substances are those that are generated in a high proportion during the above-mentioned process of reducing the molecular weight of the aromatic polyester, and are readily subjected to decomposition by the activated sludge prepared in the first step.

In the present invention, potassium ions are added to the activated sludge in the second step.

The present inventors have diligently investigated a method for treating wastewater containing decomposition products of aromatic polyesters such as PET, for example, wastewater (liquid to be treated) generated in a chemical recycling process of aromatic polyesters. As a result, they have discovered that, by adding potassium ions to the activated sludge in an appropriate amount, it is possible to rapidly treat wastewater (liquid to be treated) containing BHET (substance to be treated), which has conventionally bean difficult to decompose, whereby completing the present invention.

The potassium-containing substance used for adding potassium ions is not particularly limited, and examples thereof include potassium chloride, potassium bromide, and potassium hydroxide. Note that seawater, artificial seawater, or industrial water may also be effectively used for adding potassium ions.

In the second step, the concentration of potassium ions in the activated sludge is preferably about 1 ppm or more and about 200 ppm or less, more preferably about 5 ppm or more and about 100 ppm or less, and still more preferably about 10 ppm or more and about 50 ppm or less. Thus, the treatment efficiency of the liquid to be treated containing the substance to be treated can be further improved.

In the second step, the mixed liquor suspended solids (MLSS) concentration in the activated sludge is preferably about 2000 mg/L or more and about 7000 mg/L or less, more preferably about 3000 mg/L or more and about 6500 mg/L or less, and still more preferably about 4000 mg/L or more and about 6000 mg/L or less. Thus, the substance to be treated can be effectively decomposed while maintaining the settling properties of the sludge, and separation of the treated water from the sludge can be smoothly performed.

In the second step, the temperature of the activated sludge is preferably about 10°C or higher and about 40°C or lower, more preferably about 15°C or higher and about 35°C or lower, and still more preferably about 20°C or higher and about 30°C or lower. Thus, decomposition of the substance to be treated by the activated sludge can be maintained at a high level.

In the second step, the dissolved oxygen (DO) concentration in the activated sludge is preferably about 0.5 mg/L or more, more preferably about 0.5 mg/L or more and about 4 mg/L or less, and still more preferably about 1 mg/L or more and about 3 mg/L or less. Thus, the activity of microorganisms contributing to the decomposition of the substance to be treated can be maintained at a high level.

In the second process, the pH of the activated sludge is preferably about 5.5 or higher and about 8.5 or lower, more preferably about 6 or higher and about 8 or lower, and still more preferably about 6.5 or higher and about 7.5 or lower. Thus, the activity of microorganisms contributing to the decomposition of the substance to be treated can be maintained at a high level. Note that the pH of the activated sludge can be controlled by adjusting the amount (aeration rate) of oxygen supplied to the mixed liquor.

In the second step, the total nitrogen concentration in the activated sludge is preferably about 1 ppm or more and about 50 ppm or less per 100 ppm of chemical oxygen demand (COD), more preferably about 3 ppm or more and about 20 ppm or less, and still more preferably about 5 ppm or more and about 15 ppm or less. Note that the total nitrogen concentration in the activated sludge can be adjusted, for example, by adding nitrates, urea, or the like to the activated sludge.

Furthermore, in the second step, the total phosphorus concentration in the activated sludge is preferably about 0.1 ppm or more and about 5 ppm or less per 100 ppm of chemical oxygen demand (COD), more preferably about 0.2 ppm or more and about 3 ppm or less, and still more preferably about 0.4 ppm or more and about 1.5 ppm or less. Note that the total phosphorus concentration in the activated sludge can be adjusted, for example, by adding phosphates or the like to the activated sludge.

By adjusting the total nitrogen concentration and/or the total phosphorus concentration in the activated sludge within the above ranges, the activity of microorganisms contributing to the decomposition of the substance to be treated can be maintained at a high level.

In the second step, the time for retaining the liquid to be treated in a storage section (activated sludge tank) storing the activated sludge is preferably about 10 hours or longer and about 100 hours or shorter, more preferably about 15 hours or longer and about 75 hours or shorter, and still more preferably about 20 hours or longer and about 50 hours or shorter. Thus, the contact time between the substance to be treated and the activated sludge (microorganisms contributing to the decomposition of the substance to be treated) can be sufficiently secured, thereby further enhancing the decomposition efficiency of the substance to be treated.

Moreover, in the second step, the concentration of the substance to be treated in the liquid to be treated is preferably about 0.01% by mass or more and about 10% by mass or less, more preferably about 0.05% by mass or more and about 5% by mass or less, and still more preferably about 0.1% by mass or more and about 1% by mass or less. Thus, the amount of the substance to be treated that is exposed to the activated sludge can be maintained at an appropriate level, thereby maintaining high decomposition efficiency of the substance to be treated.

The decomposition efficiency (decomposition capability) of the substance to be treated by the activated sludge can be determined by measuring the amount of the substance to be treated remaining in the liquid to be treated after the decomposition treatment, for example, by thin-layer chromatography (TLC), high-performance liquid chromatography (HPLC), or the like.

The wastewater treatment device of the present invention includes an activated sludge tank for storing activated sludge and is configured to perform the activated sludge-based decomposition treatment method as described above. According to such a wastewater treatment device, even wastewater containing a high concentration of PET degradation products can be effectively decomposed.

The activated sludge of the present invention is used for decomposing a substance to be treated in a mixed liquor obtained by mixing the activated sludge with a liquid to be treated containing the substance to be treated, the substance to be treated including at least one selected from phthalic acids, esters thereof, and oligomers thereof. The activated sludge is obtained from a wastewater treatment device configured to treat wastewater generated in a process of reducing the molecular weight of an aromatic polyester, and potassium ions are added to the activated sludge. As described above, by adding potassium ions to the activated sludge in an appropriate amount, it is possible to rapidly treat wastewater (liquid to be treated) containing BHET (substance to be treated), which has conventionally been difficult to decompose.

The concentration of the potassium ions in the activated sludge is preferably about 1 ppm or more and about 200 ppm or less, more preferably about 5 ppm or more and about 100 ppm or less, and still more preferably about 10 ppm or more and about 50 ppm or less. Thus, the treatment efficiency of the liquid to be treated containing the substance to be treated can be further enhanced.

It should be noted that other details of the activated sludge and the substance to be treated may be the same as or similar to those described above for the activated sludge-based decomposition treatment method.

Furthermore, the present invention may also be provided in the following aspects.
(1) An activated sludge-based decomposition treatment method comprising: a first step of preparing activated sludge in a wastewater treatment apparatus configured to treat wastewater generated in a process of reducing the molecular weight of an aromatic polyester; and a second step of decomposing a substance to be treated in a mixed liquor obtained by mixing the activated sludge with a liquid to be treated containing the substance to be treated, the substance to be treated including at least one selected from phthalic acids, esters thereof, and oligomers thereof, wherein, in the second step, potassium ions are added to the activated sludge.
(2) The activated sludge-based decomposition treatment method according to (1), wherein: in the second step, the concentration of the potassium ions in the activated sludge is 1 ppm or more and 200 ppm or less.
(3) The activated sludge-based decomposition treatment method according to (1) or (2), wherein: in the second step, the mixed liquor suspended solids (MLSS) concentration in the activated sludge is 2000 mg/L or more and 7000 mg/L or less.
(4) The activated sludge-based decomposition treatment method according to any one of (1) to (3), wherein: in the second step, the temperature of the activated sludge is 10°C or higher and 40°C or lower.
(5) The activated sludge-based decomposition treatment method according to any one of (1) to (4), wherein: in the second step, the dissolved oxygen concentration in the activated sludge is 0.5 mg/L or more.
(6) The activated sludge-based decomposition treatment method according to any one of (1) to (5), wherein: in the second step, the pH of the activated sludge is 5.5 or higher and 8.5 or lower.
(7) The activated sludge-based decomposition treatment method according to any one of (1) to (6), wherein: in the second step, the total nitrogen concentration in the activated sludge is 1 ppm or more and 50 ppm or less per 100 ppm of chemical oxygen demand (COD).
(8) The activated sludge-based decomposition treatment method according to any one of (1) to (7), wherein: in the second step, the total phosphorus concentration in the activated sludge is 0.1 ppm or more and 5 ppm or less per 100 ppm of chemical oxygen demand (COD).
(9) The activated sludge-based decomposition treatment method according to any one of (1) to (8), wherein: in the second step, the time for retaining the liquid to be treated in a storage section storing the activated sludge is 10 hours or longer and 100 hours or shorter.
(10) The activated sludge-based decomposition treatment method according to any one of (1) to (9), wherein: in the second step, the concentration of the substance to be treated in the liquid to be treated is 0.01% by mass or more and 10% by mass or less.
(11) The activated sludge-based decomposition treatment method according to any one of (1) to (10), wherein: the liquid to be treated is generated in a process of chemically recycling an aromatic polyester.
(12) The activated sludge-based decomposition treatment method according to any one of (1) to (11), wherein: the liquid to be treated contains, as the substance to be treated, mainly bis(2-hydroxyethyl) terephthalate (BHET).
(13) A wastewater treatment apparatus comprising: an activated sludge tank configured to store activated sludge, wherein the wastewater treatment apparatus is configured to perform the activated sludge-based decomposition treatment method according to any one of (1) to (12).
(14) Activated sludge used for decomposing a substance to be treated in a mixed liquor obtained by mixing the activated sludge with a liquid to be treated containing the substance to be treated, the substance to be treated including at least one selected from phthalic acids, esters thereof, and oligomers thereof, wherein: the activated sludge is obtained from a wastewater treatment apparatus configured to treat wastewater generated in a process of reducing the molecular weight of an aromatic polyester, and potassium ions are added to the activated sludge.
(15) The activated sludge according to (14), wherein: the concentration of the potassium ions in the activated sludge is 1 ppm or more and 200 ppm or less.

Needless to say, the present invention is not limited to the above.

As described above, although various embodiments according to the present invention have been described, they are presented by way of example and are not intended to limit the scope of the invention. The novel embodiments may be implemented in various other forms, and various omissions, substitutions, and changes may be made without departing from the spirit of the invention. These embodiments and their modifications are intended to be included within the scope and spirit of the invention, as well as within the scope of the invention described in the claims and their equivalents.

### [Examples]

Hereinafter, the present invention will be described in more detail using Examples. Note that the present invention is not limited to these Examples.

### 1. Preparation

### 1-1. Preparation of BHET, Activated Sludge, Seawater, and Artificial Seawater

- BHET: BHET manufactured by PET Refine Technology Co., Ltd.
- Activated sludge: Activated sludge collected from an activated sludge tank of a wastewater treatment device (a wastewater treatment device configured to treat wastewater discharged in a chemical recycling process of PET) at the Kitakyushu Hibikinada Plant of JEPLAN, Inc.
- Seawater: Seawater collected in Shimoda City, Shizuoka Prefecture
- Artificial Seawater: "MARINE ART SF-1" manufactured by Osaka Yakken Co., Ltd.

The artificial seawater was prepared by adding distilled water to a composition containing the individual components in the amounts indicated in Table 1 so that the total volume became 1000 mL.

**[Table 1]**

| Component | Amount | Unit |
|---|---|---|
| NaCl | 22.1 | g |
| KCl | 0.61 | g |
| Na₂SO₄ | 3.9 | g |
| MgCl₂·6H₂O | 9.9 | g |
| CaCl₂·2H₂O | 1.5 | g |

### 1-2. Demineralization Treatment of Activated Sludge

### Demineralization treatment was performed on the activated sludge.

Specifically, first, 50 mL of the activated sludge was placed in a centrifuge tube and centrifuged under the conditions of 700 × g for 1 minute at room temperature to separate it into an activated sludge layer and a supernatant. Next, after removing 40 mL of the supernatant, 40 mL of deionized water was added to the centrifuge tube, and mixed to uniformly disperse the contents. This series of operations was repeated three times.

### 1-3. Preparation of Activation Solution and Liquid to Be Treated

The activation solution was prepared by adding 1.2 g of BHET (substance to be treated) to 270 mL of deionized water, heating to dissolve the BHET, and then adding 30 mL of a 1 M phosphate buffer solution in which 1.2 g of ammonium sulfate had been dissolved. The concentration of BHET in the activation solution was about 0.4% by mass.

The liquid to be treated was prepared by adding 0.6 g of BHET (substance to be treated) to 150 mL of deionized water, heating to dissolve the BHET, and then adding 150 mL of a 1 M phosphate buffer solution in which 0.6 g of ammonium sulfate had been dissolved. The concentration of BHET in the liquid to be treated was about 0.2% by mass.

### 2. Decomposition Treatment Experiment (Example 1)

First, 15 mL of the demineralized activated sludge, 30 µL of a 1% by mass potassium chloride aqueous solution, 270 µL of deionized water, and 15 mL of the activation solution were added to a Sakaguchi flask, and the mixture was stirred with a magnetic stirring bar at a rotational speed of 135 rpm and treated at 30°C for 2 days.

Next, after removing 15 mL of the treated liquid, 15 mL of the activation solution, 30 µL of a 1% by mass potassium chloride aqueous solution, and 270 µL of deionized water were newly added, and the mixture was stirred with a magnetic stirring bar at a rotational speed of 135 rpm and further treated at 30°C for additional 2 days.

Next, a mixed liquor obtained by adding 15 mL of the treated liquid after 4 days of treatment to 15 mL of the liquid to be treated was stirred with a magnetic stirring bar at a rotational speed of 160 rpm and treated at 30°C for 3 hours.

Note that the concentration of potassium ions in the treated liquid after 4 days of treatment was about 8 ppm, and the pH was about 7.

After 3 hours, 0.5 ml of the treated liquid was sampled, 0.5 ml of acetonitrile for HPLC was added, and the mixture was used as a sample for HPLC measurement.

Note that the above process was performed twice to prepare two samples for HPLC measurement.

### (Example 2)

Decomposition treatment was performed in the same manner as in Example 1, except that 150 µL of a 1% by mass potassium chloride aqueous solution and 150 µL of deionized water were used instead of 30 µL of the 1% by mass potassium chloride aqueous solution and 270 µL of the deionized water.

Note that the concentration of potassium ions in the treated liquid after 4 days of treatment was about 40 ppm, and the pH was about 7.

### (Example 3)

Decomposition treatment was performed in the same manner as in Example 1, except that 300 µL of a 1% by mass potassium chloride aqueous solution was used instead of 30 µL of the 1% by mass potassium chloride aqueous solution and 270 µL of the deionized water.

Note that the concentration of potassium ions in the treated liquid after 4 days of treatment was about 80 ppm, and the pH was about 7.

### (Example 4)

Decomposition treatment was performed in the same manner as in Example 1, except that 500 µL of the seawater (potassium ion concentration: about 400 ppm) was used instead of 30 µL of the 1% by mass potassium chloride aqueous solution and 270 µL of the deionized water.

Note that the concentration of potassium ions in the treated liquid after 4 days of treatment was about 10 ppm, and the pH was about 7.

### (Example 5)

Decomposition treatment was performed in the same manner as in Example 1, except that 500 µL of the artificial seawater (potassium ion concentration: about 300 ppm) was used instead of 30 µL of the 1% by mass potassium chloride aqueous solution and 270 µL of the deionized water.

Note that the concentration of potassium ions in the treated liquid after 4 days of treatment was about 8 ppm, and the pH was about 7.

### (Example 6)

Decomposition treatment was performed in the same manner as in Example 1, except that, instead of 270 µL of the deionized water, 75 µL of a 10% by mass sodium chloride aqueous solution, 60 µL of a 5% by mass magnesium sulfate aqueous solution, and 30 µL of a 5% by mass calcium chloride aqueous solution were used.

Note that the concentration of potassium ions in the treated liquid after 4 days of treatment was about 8 ppm, and the pH was about 7.

### (Comparative Example 1)

Decomposition treatment was performed in the same manner as in Example 1, except that 75 µL of a 10% by mass sodium chloride aqueous solution and 225 µL of deionized water were used instead of 30 µL of the 1% by mass potassium chloride aqueous solution and 270 µL of the deionized water.

Note that the concentration of potassium ions in the treated liquid after 4 days of treatment was about 0 ppm, and the pH was about 7.

### (Comparative Example 2)

Decomposition treatment was performed in the same manner as in Example 1, except that 60 µL of a 5% by mass sodium chloride aqueous solution and 240 µL of deionized water were used instead of 30 µL of the 1% by mass potassium chloride aqueous solution and 270 µL of the deionized water.

Note that the concentration of potassium ions in the treated liquid after 4 days of treatment was about 0 ppm, and the pH was about 7.

### (Comparative Example 3)

Decomposition treatment was performed in the same manner as in Example 1, except that 30 µL of a 5% by mass sodium chloride aqueous solution and 270 µL of the deionized water were used instead of 30 µL of the 1% by mass potassium chloride aqueous solution and 270 µL of the deionized water.

Note that the concentration of potassium ions in the treated liquid after 4 days of treatment was about 0 ppm, and the pH was about 7.

### (Comparative Example 4)

Decomposition treatment was performed in the same manner as in Example 1, except that 30 µL of the 1% by mass potassium chloride aqueous solution and 270 µL of the deionized water were omitted.

Note that the concentration of potassium ions in the treated liquid after 4 days of treatment was about 0 ppm, and the pH was about 7.

### (Reference Sample)

First, 15 mL of the activation solution was added to 15 mL of deionized water and thoroughly mixed. Then, 15 mL of the mixture was taken, and 15 mL of the activation solution was newly added thereto and thoroughly mixed.

Next, 15 mL of the resulting mixed liquor was taken, 15 mL of the liquid to be treated was added thereto, and the mixture was thoroughly mixed.

Finally, 0.5 mL of the resulting mixed liquor was taken, 0.5 mL of acetonitrile for HPLC was added thereto, and the resulting mixture was used as a reference HPLC measurement sample.

### 2. Measurement

### 2-1. Measurement of Decomposition Rates of BHE, MHET, and TPA

To 0.1 mL of the HPLC measurement sample obtained in each Example and Comparative Example, or to 0.1 mL of the reference HPLC measurement sample, a mobile phase (formic acid/acetonitrile/water = 1/2/7 by volume) was added to dilute the sample tenfold, and the mixture was stirred, filtered through a 0.2 µm filter, and then subjected to HPLC analysis.

For the HPLC analysis, "LC-2010A HT" manufactured by Shimadzu Corporation was used as the device, and "COSMOCIL 5C18-AR-II (4.6 ID × 250 mm)" and "5C18-AR-II guard column", both manufactured by Nacalai Tesque, Inc., were used as the columns.

The analysis was performed under isocratic elution conditions at a flow rate of 1 mL/min and a column temperature of 40°C using the above mobile phase, and detection was performed at a wavelength of 254 nm.

Based on the retention times, peaks of BHET, MHET, and TPA contained in the HPLC measurement samples were identified, and the peak areas were calculated.

For BHET, a calibration curve for BHET concentration and peak area was prepared using a BHET aqueous solution dissolved at 70°C, and quantitative performance was also verified.

The results are summarized in Table 2. 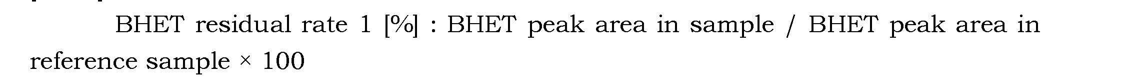 BHET residual rate 1 [%] : BHET peak area in sample / BHET peak area in reference sample × 100 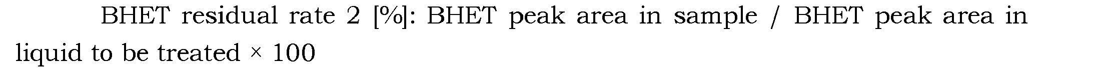 BHET residual rate 2 [%]: BHET peak area in sample / BHET peak area in liquid to be treated × 100

The numerical values in Table 2 represent the average of two samples.

**[Table 2]**

| | Ex. 1 | Ex. 2 | Ex. 3 | Ex. 4 | Ex. 5 | Ex. 6 | Comp. Ex. 1 | Comp. Ex. 2 | Comp. Ex. 3 | Comp. Ex. 4 |
|---|---|---|---|---|---|---|---|---|---|---|
| BHET residual rate 1 [%] | - | 27 | - | 28 | 28 | 27 | 37 | 38 | 37 | 37 |
| BHET residual rate 2 [%] | - | 56 | - | 56 | 57 | 55 | 77 | 77 | 76 | 75 |
| BHET residual rate 1 [%] | 33 | 28 | 31 | 35 | 35 | - | - | - | - | 40 |
| BHET residual rate 2 [%] | 75 | 65 | 72 | 82 | 82 | - | - | - | - | 92 |

Moreover, the residual rates of MHET and TPA in the sample obtained in each Example were 70% or less relative to those of the MHET and TPA in the reference sample.

Note that, in each of Examples 1 to 6, the mixed liquor suspended solids (MLSS) concentration in the treated liquid after 4 days of treatment is preferably 2000 mg/L or more and 7000 mg/L or less, the dissolved oxygen concentration is preferably 0.5 mg/L or more, the total nitrogen concentration is preferably 1 ppm or more and 50 ppm or less per 100 ppm of chemical oxygen demand (COD), and the total phosphorus concentration is preferably 0.1 ppm or more and 5 ppm or less per 100 ppm of chemical oxygen demand (COD).

Furthermore, when the activated sludge stored in the storage section of the wastewater treatment device is subjected to the same pretreatment as in Examples 1 to 6, and the liquid to be treated is then supplied to the storage section to perform decomposition treatment of BHET, the retention time of the liquid to be treated in the storage section is preferably 10 hours or more and 100 hours or less.

## Claims

1. An activated sludge-based decomposition treatment method comprising:
a first step of preparing activated sludge in a wastewater treatment apparatus configured to treat wastewater generated in a process of reducing the molecular weight of an aromatic polyester; and
a second step of decomposing a substance to be treated in a mixed liquor obtained by mixing the activated sludge with a liquid to be treated containing the substance to be treated, the substance to be treated including at least one selected from phthalic acids, esters thereof, and oligomers thereof,
wherein, in the second step, potassium ions are added to the activated sludge.

2. The activated sludge-based decomposition treatment method according to Claim 1, wherein:
in the second step, the concentration of the potassium ions in the activated sludge is 1 ppm or more and 200 ppm or less.

3. The activated sludge-based decomposition treatment method according to Claim 1 or Claim 2, wherein:
in the second step, the mixed liquor suspended solids (MLSS) concentration in the activated sludge is 2000 mg/L or more and 7000 mg/L or less.

4. The activated sludge-based decomposition treatment method according to any one of Claims 1 to 3, wherein:
in the second step, the temperature of the activated sludge is 10°C or higher and 40°C or lower.

5. The activated sludge-based decomposition treatment method according to any one of Claims 1 to 4, wherein:
in the second step, the dissolved oxygen concentration in the activated sludge is 0.5 mg/L or more.

6. The activated sludge-based decomposition treatment method according to any one of Claims 1 to 5, wherein:
in the second step, the pH of the activated sludge is 5.5 or higher and 8.5 or lower.

7. The activated sludge-based decomposition treatment method according to any one of Claims 1 to 6, wherein:
in the second step, the total nitrogen concentration in the activated sludge is 1 ppm or more and 50 ppm or less per 100 ppm of chemical oxygen demand (COD).

8. The activated sludge-based decomposition treatment method according to any one of Claims 1 to 7, wherein:
in the second step, the total phosphorus concentration in the activated sludge is 0.1 ppm or more and 5 ppm or less per 100 ppm of chemical oxygen demand (COD).

9. The activated sludge-based decomposition treatment method according to any one of Claims 1 to 8, wherein:
in the second step, the time for retaining the liquid to be treated in a storage section storing the activated sludge is 10 hours or longer and 100 hours or shorter.

10. The activated sludge-based decomposition treatment method according to any one of Claims 1 to 9, wherein:
in the second step, the concentration of the substance to be treated in the liquid to be treated is 0.01% by mass or more and 10% by mass or less.

11. The activated sludge-based decomposition treatment method according to any one of Claims 1 to 10, wherein:
the liquid to be treated is generated in a process of chemically recycling an aromatic polyester.

12. The activated sludge-based decomposition treatment method according to any one of Claims 1 to 11, wherein:
the liquid to be treated contains, as the substance to be treated, mainly bis(2-hydroxyethyl) terephthalate (BHET).

13. A wastewater treatment apparatus comprising:
an activated sludge tank configured to store activated sludge,
wherein the wastewater treatment apparatus is configured to perform the activated sludge-based decomposition treatment method according to any one of Claims 1 to 12.

14. Activated sludge used for decomposing a substance to be treated in a mixed liquor obtained by mixing the activated sludge with a liquid to be treated containing the substance to be treated, the substance to be treated including at least one selected from phthalic acids, esters thereof, and oligomers thereof, wherein:
the activated sludge is obtained from a wastewater treatment apparatus configured to treat wastewater generated in a process of reducing the molecular weight of an aromatic polyester, and potassium ions are added to the activated sludge.

15. The activated sludge according to Claim 14, wherein:
the concentration of the potassium ions in the activated sludge is 1 ppm or more and 200 ppm or less.
